# EUROPEAN PATENT SPECIFICATION

(11) **EP 0 494 732 B1**
(45) Date of publication and mention of the grant of the patent: **10.05.1995**
(21) Application number: 92300035.0
(22) Date of filing: 03.01.1992
(51) Int. Cl.: A61M 25/00

(54) **Catheter with curved distal end**
Katheter mit gekrümmtem distalen Ende
Cathéter avec extrémité distal courbé

(30) Priority: 07.01.1991 US 638376
(43) Date of publication of application: 15.07.1992
(73) Proprietor: E-Z-EM, INC., Westbury New York 11590 (US)
(72) Inventor: Hobbs, Eamonn, New York, N.Y. 12804 (US); Appling, William M., New York, N.Y. 12838 (US); Hawkins, Irvin F., Micanopy, Florida (US)
(74) Representative: Jennings, Nigel Robin

(56) References cited:
- EP-A- 0 154 403
- EP-A- 0 346 012
- US-A- 3 999 554
- US-A- 4 169 464

## Description

The present invention relates to a catheter, e.g. for use in angiographic procedures, and more particularly to such a catheter which is useable for delivery of contrast media at appropriate flow rates and pressures. The invention is concerned with catheters of so called pigtail type having a proximal portion, a distal portion and a central axis, the distal portion having at least one opening formed therein and including a curved portion, which defines a zone substantially encircling the central axis, and a free end portion.

Angiographic catheters are provided in four basic shapes: straight, single curve, multiple curve, and pigtail. Pigtail catheters permit an uneven distribution of rapidly injected contrast media while allowing end hole and side hole jetting. Uses for pigtail catheters include aortography, arteriography, and angiography. To introduce a pigtail catheter into a vessel a guide wire is used to straighten the catheter. The catheter is formed of a resilient material so that the catheter can resume its normal shape when the guide wire is removed.

When a contrast medium is ejected from a catheter at a high velocity, the stream of medium ejected may cause damage to the artery or vein being studied. Such damage occurs when the high velocity stream displaces plaque in the vessel or perforates the vessel wall. Additionally, damage can be caused to the vessel wall by contact with the catheter tip itself, and the catheter tip in turn may be damaged by such contact.

During high pressure injection pigtail catheters can uncoil at the tip, turning the pigtail into a hook shape, which can send a jetting stream of contrast medium directly at the vessel wall. Also during high pressure injection the catheter tip may flap around allowing the uncoiled tip to impinge violently against the vessel wall, thereby causing trauma.

When performing angiographic studies, it is preferable to use as little contrast medium as possible. This maximises patient safety while minimising the expense of the procedure.

It is an object of the present invention to provide a catheter which is capable of delivering a contrast medium at high pressure into the vascular system while protecting the vascular system from injury.

Another object of the present invention is the provision of such a catheter which minimises the amount of contrast medium needed to perform a study.

Yet a further object of the present invention is the provision of such a catheter whose tip is protected from inadvertent contact with a vessel wall.

Yet a further object of the present invention is the provision of such a catheter whose openings are positioned to prevent the exiting contrast medium jet from impinging against the artery wall, thereby reducing any chance of jet-induced trauma.

EP-A-0 154 403 is the closest prior art, on which the preamble is based.

According to the present invention a catheter of the type referred to above is characterised in that a first opening is formed in the free end portion and that the first opening is situated substantially on the central axis and is directed in the axial direction, that a plurality of inwardly directed additional openings is formed in the curved portion and that the catheter has no opening which is outwardly directed. Thus, in use, the curved portion centres the catheter within the blood vessel and causes the free end portion and, more particularly, the opening formed therein to be spaced from the wall of the blood vessel, that is to say the artery or vein. Due to the fact that the opening is directed substantially in the axial direction, a jet of contrast medium discharged from it cannot impinge directly against the blood vessel wall whereby the blood vessel is protected from damage and the risk of trauma is reduced. Significant movement of the tip of the catheter is prevented by the engagement of the curved portion with the blood vessel wall.

Furthermore, the flow of contrast medium through the additional openings will impinge against the axial flow through the first opening whereby the latter flow is located and stabilised. For the latter purpose it is preferred that the first opening is situated at a distance from the proximal end of the catheter which is between that of the additional openings which are nearest and closest, respectively, to the proximal end of the catheter.

In the preferred embodiment the free end portion includes a terminal portion which is substantially coaxial with the central axis and the first opening is formed in the free end of the terminal portion. The first opening is preferably directed towards the proximal portion.

Whilst the curved portion of the catheter is normally curved, it is of course desirable that it may be temporarily straightened to facilitate its insertion into a blood vessel and it is preferred that at least the curved portion and preferably the entire catheter is made of resilient material, e.g. nylon, whereby after the curved portion has been temporarily straightened it reassumes its normal curved configuration due to its resilience.

Further features and details of the invention will be apparent from the following description of one specific embodiment which is given by way of example with reference to the accompanying drawings, in which:-
Figure 1 is a side view of a self-centering angiographic catheter in accordance with the present invention, with the middle segment of the catheter omitted;
Figure 2 is an enlarged view of the distal portion of the catheter shown in Figure 1; and
Figure 3 is an end view of the distal portion of the catheter shown in Figure 1.

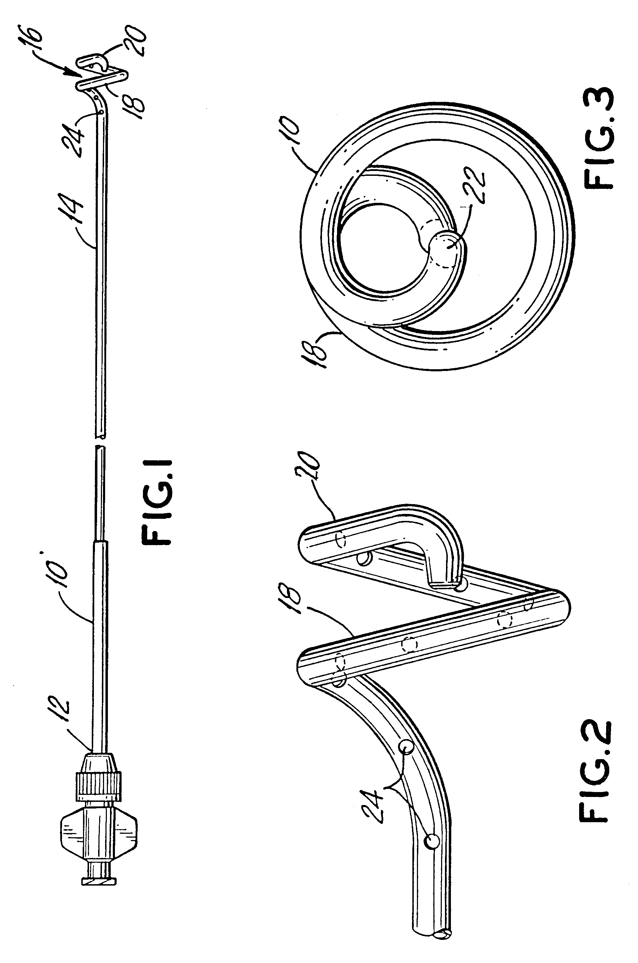

Referring now to the drawings, the catheter 10 has a proximal end 12, a distal end 16 and a central axis.

The catheter 10 includes a distal segment 14 having a normally curved portion 18 and an end portion 20, in whose free end a first end opening 22 is formed.

The curved portion 18 is generally helical and defines a zone which substantially encircles the central axis of the catheter. The catheter terminates in the end portion 20 which extends from the curved portion 18 to a position substantially on the axis of the catheter where it is directed axially towards the proximal end 12. More specifically, the end portion 20 includes a portion which extends from the curved portion 18 generally to the axis of the catheter and a terminal portion which extends generally along the axis and has the opening 22 in its end which is directed along the axis. In use, the curved portion 18 centres the catheter 10 in the lumen of the vessel being studied and, more specifically, the curved portion 18 centres the end portion 20 and first opening 22 such that inadvertent contact between the vessel wall and the end portion 20 is avoided. This prevents damage to either the catheter end portion 20 or to the vessel wall. Because the tip is pointing axially the contrast medium bolus is much tighter, hence less contrast medium will be used.

In the illustrated preferred embodiment, the curved portion 18 is formed with a plurality of additional openings 24 therein which are all positioned such that they face inwardly. This positioning of the additional openings 24, in conjunction with the centering of the first opening 22, directs the stream of contrast medium towards the centre of the vessel where the blood absorbs its energy before the high velocity stream reaches the vessel wall. This reduces the chance of contrast extravasation or embolism due to displaced plaque.

In order to introduce the catheter into a vessel it is necessary to straighten the normally curved portion 18. After introduction, the normally curved portion must be capable of reassuming its curved configuration. Thus the catheter is formed of a material, such as nylon, with sufficient resiliency to permit this to occur.

In the preferred embodiment, when the catheter is in its coiled configuration the first opening 22, and the additional openings 24, are positioned within about one centimetre of each other. This produces a tight bolus of contrast medium and reduces the amount of contrast medium needed in order to do a study. In the preferred embodiment there are ten additional openings 24. These may be holes, slits or any other appropriate openings.

## Claims

1. A tubular catheter for use in a blood vessel, the catheter having a proximal portion, a distal portion and a central axis, the distal portion having at least one opening formed therein and including a curved portion, which defines a zone substantially encircling the central axis, and a free end portion, characterised in that a first opening (22) is formed in the free end portion (20) and that the first opening (22) is situated substantially on the central axis and is directed in the axial direction, that a plurality of inwardly directed additional openings (24) is formed in the curved portion (18) and that the catheter has no opening which is outwardly directed.

2. A catheter as claimed in claim 1 characterised in that the free end portion (20) includes a terminal portion which is substantially coaxial with the central axis and that the first opening (22) is formed in the free end of the terminal portion.

3. A catheter as claimed in claim 1 characterised in that the first opening (22) is directed towards the proximal portion (10).

4. A catheter as claimed in any one of the preceding claims characterised in that at least the curved portion (18) is formed of resilient material, e.g. nylon, whereby it my be temporarily straightened and thereafter reassumes its normal curved configuration due to its resilience.

5. A catheter as claimed in any one of the preceding claims characterised in that the distance between the openings (22,24) does not exceed about one centimetre when the curved portion (18) is in its normal curved configuration.

## Patentansprüche

1. Rohrförmiger Katheter zur Verwendung in einem Blutgefäß, welcher Katheter einen proximalen Teil, einen distalen Teil und eine Mittelachse aufweist, wobei der distale Teil mindestens eine darin ausgebildete Öffnung besitzt und einen gekrümmten Abschnitt, der eine die Mittelachse im wesentlichen umgebende Zone bildet, sowie einen freien Endabschnitt enthält, dadurch gekennzeichnet, daß in dem freien Endabschnitt (20) eine erste Öffnung (22) ausgebildet ist, daß die erste Öffnung (22) im wesentlichen auf der Mittelachse liegt und in Axialrichtung gerichtet ist, daß in dem gekrümmten Abschnitt (18) mehrere nach innen gerichtete zusätzliche Öffnungen (24) ausgebildet sind, und daß der Katheter keine nach außen gerichtete Öffnung besitzt.

2. Katheter nach Anspruch 1, dadurch gekennzeichnet daß der freie Endabschniff (20) ein mit der Mittelachse im wesentlichen koaxiales Endstück enthält, und daß die erste Öffnung (22) in dem freien Ende des Endstücks ausgebildet ist.

3. Katheter nach Anspruch 1, dadurch gekennzeichnet, daß die erste Öffnung (22) gegen den proximalen Teil (10) gerichtet ist.

4. Katheter nach irgendeinem der vorhergehenden Ansprüche, dadurch gekennzeichnet, daß zumindest der gekrümmte Abschnitt (18) aus elastischem Material, z.B. Nylon, geformt ist, wodurch er vorübergehend geradegerichtet werden kann und danach aufgrund seiner Elastizität wieder seine normale, gekrümmte Konfiguration annimmt.

5. Katheter nach irgendeinem der vorhergehenden Ansprüche, dadurch gekennzeichnet, daß der Abstand zwischen den Öffnungen (22,24) etwa 1 cm nicht überschreitet, wenn der gekrümmte Abschnitt (18) sich in seiner normalen, gekrümmten Konfiguration befindet.

## Revendications

1. Cathéter tubulaire destiné à être utilisé dans un vaisseau sanguin, le cathéter ayant une partie proximale, une partie distale et un axe central, la partie distale ayant au moins une ouverture qui y est formée et comprenant une partie courbe, qui définit une zone entourant sensiblement l'axe central, et une partie d'extrémité libre, caractérisé en ce qu'une première ouverture (22) est formée dans la partie d'extrémité libre (20) et en ce que la première ouverture (22) est située sensiblement sur l'axe central et est dirigée dans la direction axiale, en ce qu'une pluralité d' ouvertures additionnelles (24) dirigées vers l'intérieur sont formées dans la partie courbe (18) et en ce que le cathéter n'a pas d'ouverture dirigée vers l'extérieur.

2. Cathéter selon la revendication 1, caractérisé en ce que la partie d' extrémité libre (20) comprend une partie terminale qui est sensiblement coaxiale à l'axe central et en ce que la première ouverture (22) est formée dans l'extrémité libre de la partie terminale.

3. Cathéter selon la revendication 1, caractérisé en ce que la première ouverture (22) est dirigée vers la partie proximale (10).

4. Cathéter selon l'une quelconque des revendications précédentes, caractérisé en ce qu'au moins la partie courbe (18) est formée d'un matériau élastique, par exemple du nylon, moyennant quoi elle peut être temporairement redressée puis reprendre ensuite sa configuration normale courbe en raison de son élasticité.

5. Cathéter selon l'une quelconque des revendications précédentes, caractérisé en ce que la distance entre les ouvertures (22,24) ne dépasse pas environ 1 cm quand la partie courbe (18) est dans sa configuration normale courbe.
